# EUROPÄISCHE PATENTSCHRIFT

(11) **EP 1 786 324 B1**
(45) Veröffentlichungstag und Bekanntmachung des Hinweises auf die Patenterteilung: **21.12.2016**
(21) Anmeldenummer: 05773956.7
(22) Anmeldetag: 24.08.2005
(51) Int. Cl.: A61B 5/117

(54) **VORRICHTUNG ZUM ERFASSEN EINES FINGERABDRUCKES**
DEVICE FOR RECORDING A DIGITAL FINGERPRINT
DISPOSITIF POUR ENREGISTRER UNE EMPREINTE DIGITALE

(30) Priorität: 08.09.2004 AT 15002004
(43) Veröffentlichungstag der Anmeldung: 23.05.2007
(73) Patentinhaber: ASMAG-Holding GmbH, 4645 Grünau im Almtal (AT)
(72) Erfinder: PADINGER, Franz, 4502 St. Marien (AT); SCHRÖTER, Klaus G., 14197 Berlin (DE)
(74) Vertreter: Ofner, Clemens
(86) Internationale Anmeldenummer: PCT/AT2005/000336
(87) Internationale Veröffentlichungsnummer: WO 2006/026794

(56) Entgegenhaltungen:
- WO-A-97/36544
- WO-A-99/39372
- WO-A-03/015189
- WO-A-2004/036484
- US-A1- 2003 090 650
- US-A1- 2004 067 393
- US-B1- 6 327 376

## Beschreibung

### Technisches Gebiet

Die Erfindung bezieht sich auf eine Vorrichtung zum Erfassen eines Fingerabdruckes mit einer lichtdurchlässigen, eine Fingerauflage bildenden Decklage, zwischen der und einer lichtemittierenden Lage eine Lage lichtempfindlicher Elemente in einer Matrixanordnung vorgesehen ist, und mit einer an die lichtempfindlichen Elemente angeschlossenen Auswerteschaltung.

### Stand der Technik

Um einen Fingerabdruck digital erfassen zu können, ohne den Fingerabdruck zunächst optisch durch ein Linsensystem abzubilden, ist es bekannt (WO 97/036544 A1), lichtempfindliche Elemente, vorzugsweise Photodioden oder Phototransistoren, in Dünnschichttechnik auf einem durchsichtigen Träger aus Glas oder Quarz anzuordnen und mit einer lichtemittierenden Lage beispielsweise aus Elektrolumineszenzdioden abzudecken. Da die lichtempfindlichen Elemente in einer Matrix reihenweise angeordnet und die Elementreihen voneinander durch lichtdurchlässige Zwischenräume voneinander getrennt sind, kann von der lichtemittierenden Lage Licht durch die lichtdurchlässigen Zwischenräume und den durchsichtigen Träger auf den Finger geworfen werden, der zum Erfassen seines Fingerabdruckes an den als Fingerauflage dienenden Träger angelegt wird. Das an den Leisten und Vertiefungen der Haut unterschiedlich reflektierte Licht wird von den lichtempfindlichen Elementen erfaßt, deren von der Stärke des reflektierten Lichtes abhängigen elektrischen Signale elementenweise in eine Auswerteschaltung zum Erstellen eines digitalen Abbildes eines Fingerabdruckes übertragen werden. Nachteilig bei dieser bekannten Vorrichtung zum Erfassen eines Fingerabdruckes ist vor allem der durch den Einsatz von lichtempfindlichen Elementen auf der Basis anorganischer Halbleiter bedingte Konstruktionsaufwand, wozu noch kommt, daß die Beleuchtung des zu erfassenden Fingerabdruckes nur durch vergleichsweise schmale Zwischenräume zwischen den lichtempfindlichen Elementen vorgenommen werden kann.

Das Dokument US 2003/090650 A1 offenbart einen Fingerabdrucksensor, der eine ebene Lichtquelle, darauf einen 2D-Bildsensor und darauf ferner eine dicht gepackte Anordnung von Faser-Elementen aufweist. Mit dieser Anordnung wird erreicht, das mit diesem Sensor sowohl ein Fingerabdruck erfasst werden kann, als auch eine Pulsoxymetrische Messung durchgeführt werden kann.

Das Dokument WO 99/39372 A2 offenbart einen Bildsensor aus organischen Halbleiterkomponenten, zur Erfassung monochromatischer, oder multi-color Abbilder. Durch selektive Anpassung der Materialkombination wird die gewünschte spektrale Empfindlichkeit in den relevanten Wellenlängenbereichen erreicht.

Da Dokument WO 03/015189 A1 offenbart einen lichtdurchlässigen Flachkörper mit zwei transparenten Deckschichten und ferner eine Aktivschicht zwischen zwei Elektrodenschichten, welche Aktivschicht im elektrischen Feld ihre Transparenz ändert. Ferner ist ein photovoltaisches Element vorgesehen, wobei die beiden Deckschichten die Aktivschicht und das photovoltaische Element einschließen.

### Darstellung der Erfindung

Der Erfindung liegt somit die Aufgabe zugrunde, eine Vorrichtung zum Erfassen eines Fingerabdruckes der eingangs geschilderten Art so auszugestalten, daß mit einfachen konstruktiven Mitteln ein digitales Abbild eines Fingerabdruckes erhalten werden kann.

Die Erfindung löst die gestellte Aufgabe dadurch, daß die Lage lichtempfindlicher Elemente eine lichtdurchlässige, photoaktive Schicht auf der Basis organischer Halbleiter zwischen zwei lichtdurchlässigen Elektrodenschichten aus einander kreuzenden Leiterbahnen aufweist. Da die lichtempfindlichen Elemente durch eine photoaktive Schicht auf der Basis organischer Halbleiter gebildet werden, kann diese photoaktive Schicht selbst lichtdurchlässig gestaltet werden, was die sonst durch die lichtundurchlässigen anorganischen Halbleiter bedingten Einschränkungen hinsichtlich der Beleuchtung des Fingerabdruckes mit Hilfe einer lichtemittierenden Lage auf der der Fingerauflage abgewandten Seite der lichtempfindlichen Elemente aufhebt. Dazu kommt ein vergleichsweise geringer Herstellungsaufwand, der einerseits auf der Löslichkeit der organischen Halbleiterwerkstoffe in herkömmlichen Lösungsmitteln und anderseits auf einer im Vergleich zu anorganischen Halbleitern geringen Beweglichkeit der Ladungsträger beruht, so daß es keiner besonderen Maßnahmen bedarf, um einzelne lichtempfindliche Bereiche voneinander abzugrenzen. Diese lichtempfindlichen Bereiche werden durch die einander kreuzenden Leiterbahnen der lichtdurchlässigen Elektrodenschichten bestimmt, weil der Ladungstransport im wesentlichen auf den Kreuzungsbereich der Leiterbahnen beschränkt ist und der Einfluß einer Ladungsbewegung zwischen benachbarten Kreuzungsbereichen der Leiterbahnen innerhalb der photoaktiven Schicht im allgemeinen vernachlässigt werden kann. Obwohl unterschiedliche organische Halbleiter zum Einsatz kommen können, werden für die photoaktive Schicht vorzugsweise zwei molekulare Komponenten eingesetzt, nämlich eine konjugierte Polymerkomponente als Elektronendonator und eine Fullerenkomponente als Elektronenakzeptor.

Wird die lichtemittierende Lage in einzelne je für sich ansteuerbare Bereiche unterteilt, so kann zunächst die für die Lichtemission erforderliche Anregungsenergie niedrig gehalten werden, weil ja der Fingerabdruck nur bereichsweise in aufeinanderfolgenden Zeitintervallen beleuchtet wird. Die bereichsweise Beleuchtung des Fingerabdruckes bietet außerdem die Möglichkeit, nur das vom Fingerabdruck reflektierte und nicht auch das emittierte Licht zu erfassen. Zu diesem Zweck werden nicht die vom emittierten Licht durchstrahlen Bereiche der photoaktiven Schicht, sondern die benachbarten Bereiche, die nur vom reflektierten Licht beaufschlagt werden, zur Auswertung ausgelesen. Werden die elektrischen Signale der sowohl vom emittierten Licht durchleuchteten als auch vom reflektierten Licht beaufschlagten Bereiche ausgewertet, so ist die Grundbelastung der photoaktiven Schicht durch das emittierte Licht zu berücksichtigen, um aufgrund der dieser Grundbelastung gegenüber gemessenen Unterschiede den Fingerabdruck zu erfassen.

Die lichtemittierende Lage kann aus Elektrolumineszenzdioden in Dünnschichttechnik aufgebaut sein. Besonders vorteilhafte Konstruktionsbedingungen ergeben sich allerdings, wenn die lichtemittierende Lage eine photoaktive Schicht auf der Basis eines organischen Halbleiters zwischen zwei Elektrodenschichten aus einander kreuzenden Leiterbahnen aufweist, von denen die Elektrodenschicht zwischen der photoaktiven Schicht und der Lage lichtempfindlicher Elemente zumindest bereichsweise lichtdurchlässig ist. In diesem Fall ergeben sich ähnliche Vorteile hinsichtlich des Aufbaus und der Ansteuerung der lichtemittierenden Lage wie bei der Lage lichtempfindlicher Elemente.

Je eine photoaktive Schicht für die lichtempfindliche und die lichtemittierende Lage kommen zum Einsatz, und zwar so, dass für die photoaktive Schicht der lichtempfindlichen Lage und die photoaktive Schicht der lichtemittierenden Lage eine gemeinsame Elektrodenschicht aus parallelen Leiterbahnen zwischen den beiden photoaktiven Schichten vorgesehen wird, was eine Konstruktionsvereinfachung mit sich bringt.

Wird die photoaktive Schicht der Lage lichtempfindlicher Elemente über eine Steuereinrichtung in zeitlicher Abhängigkeit von der Ansteuerung der lichtemittierenden Lage angesteuert, so kann ebenfalls die Erfassung des Durchlichtes durch die lichtempfindlichen Elemente für die Erfassung des Fingerabdruckes unterdrückt werden, wenn die zeitliche Abhängigkeit des Auslesens des Anregezustandes der einzelnen lichtempfindlichen Bereiche der photoaktiven Schicht von der Ansteuerung der lichtemittierenden Lage so gewählt wird, daß nicht das Durchlicht, sondern erst das am Fingerabdruck reflektierte Licht ausgewertet wird.

### Kurze Beschreibung der Zeichnung

In der Zeichnung ist der Erfindungsgegenstand beispielsweise dargestellt. Es zeigen
Fig. 1 eine Vorrichtung zum Erfassen eines Fingerabdruckes in einem schematischen Querschnitt und
Fig. 2 die Vorrichtung nach der Fig. 1 in einer schematischen, zum Teil aufgerissenen Draufsicht.

### Weg zur Ausführung der Erfindung

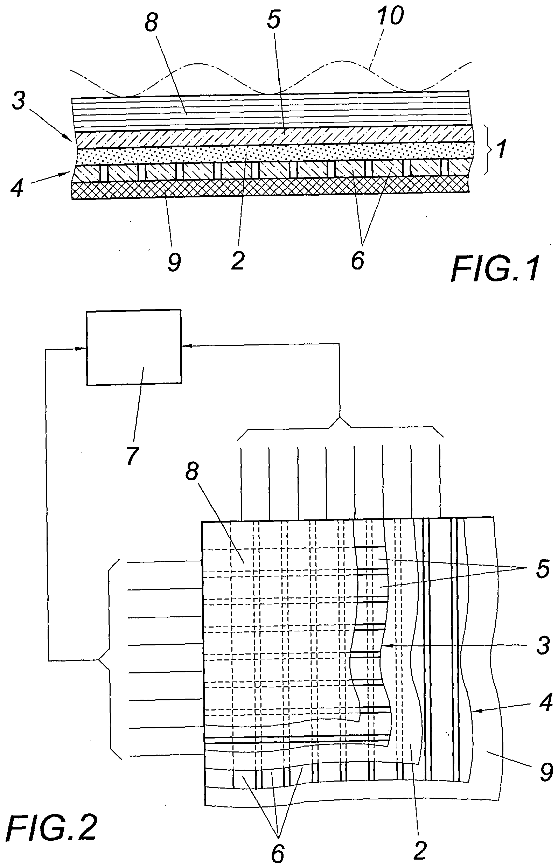

Gemäß dem dargestellten Ausführungsbeispiel weist die Vorrichtung zum Erfassen eines Fingerabdruckes eine Lage 1 lichtempfindlicher Elemente in Form einer lichtdurchlässigen, photoaktiven Schicht 2, beispielsweise aus zwei molekularen organischen Komponenten, nämlich einer konjugierten Polymerkomponente als Elektronendonator und einer Fullerenkomponente als Elektronenakzeptor, auf. Diese photoaktive Schicht ist zwischen Elektrodenschichten 3, 4 aus einander kreuzenden Leiterbahnen 5, 6 vorgesehen, die an eine Auswerteschaltung 7 angeschlossen sind, wie dies in der Fig. 2 angedeutet ist. Die Leiterbahnen 5 der als lochsammelnde Kathode wirksamen Elektrodenschicht 3 bestehen vorteilhaft aus einem Indium/Zinn-Oxid (ITO) während als elektronensammelnde Elektrode die Elektrodenschicht 4 Leiterbahnen aus Aluminium aufweist. Zwischen diesen Elektrodenschichten 3 und 4 und der photoaktiven Schicht 2 können zusätzlich Polymerschichten zur Verbesserung des Loch- bzw. Elektronenübergangs vorgesehen sein. Die photoaktive Schicht 2 mit den Elektrodenschichten 3, 4 ist auf einer lichtdurchlässigen Decklage 8 aufgebracht, die zugleich eine Fingerauflage bildet. Auf der von der Decklage 8 abgewandten Seite der Lage 1 lichtempfindlicher Elemente ist eine lichtemittierende Lage 9 vorgesehen, die beispielsweise aus organischen Leuchtemissionsdioden oder Elektrolumineszenzdioden aufgebaut sein kann.

Um die durch rillenförmige Vertiefungen voneinander getrennten Hautleisten eines in der Fig. 1 strichpunktiert angedeuteten Fingerabdruckes 10 zu erfassen, wird die lichtemittierende Lage 9 angeregt. Das emittierte Licht durchdringt die lichtdurchlässige Lage 1 lichtempfindlicher Elemente sowie die Decklage 8 und wird am Fingerabdruck 10 des an der Decklage 8 anliegenden Fingers reflektiert, um die Lage 1 lichtempfindlicher Elemente zu beaufschlagen. Wird die Anordnung so getroffen, daß die lichtempfindlichen Elemente, die in der photoaktiven Schicht 2 durch die Kreuzungsbereiche der Leiterbahnen 5 und 6 bestimmt werden, sowohl vom emittierten Licht zur Beleuchtung des Fingerabdruckes 10 als auch vom am Fingerabdruck 10 reflektierten Licht beaufschlagt werden, wie dies z.B. bei einer vollflächigen Beleuchtung der Fall ist, so ist bei der Auswertung der elektrischen Signale die durch das emittierte Licht gegebene Grundbelastung der lichtempfindlichen Elemente zu berücksichtigen, so daß nur die sich gegenüber dieser Grundbelastung ergebenden Unterschiede der elektrischen Signale für die Erfassung des Fingerabdruckes 10 herangezogen werden können. Soll lediglich die Beaufschlagung der lichtempfindlichen Elemente durch das reflektierte Licht für die Erfassung des Fingerabdruckes 10 ausgewertet werden, so stehen zwei Möglichkeiten offen. Es können entweder der Fingerabdruck 10 in aufeinanderfolgenden Zeitintervallen nur bereichsweise beleuchtet werden, um das reflektierte Licht über die lichtempfindlichen Elemente in anschließenden Bereichen zu erfassen, oder das Emittieren des Lichtes und der Empfang des reflektierten Lichtes zeitlich gestaffelt werden, so daß die lichtempfindlichen Elemente nach dem Beenden der Lichtemission während des Empfangs des reflektierten Lichtes ausgelesen werden. Für die bereichsweise Ansteuerung der lichtemittierenden Lage 9 ist die Lage 9 entsprechend aufzubauen und anzusteuern, wobei die Lage 9 ähnlich der Lage 1 der lichtempfindlichen Elemente eine photoaktive Schicht auf der Basis organischer Halbleiter zwischen zwei Elektrodenschichten aus einander kreuzenden Leiterbahnen aufweisen kann, in deren Kreuzungsbereich sich die einzeln ansteuerbaren lichtemittierenden Elemente ergeben. Über die Leiterbahnen können somit einzelne lichtemittierende Elemente ausgewählt und mit elektrischer Energie zur Abgabe einer Lichtstrahlung versorgt werden. In Abhängigkeit von der Auswahl der lichtemittierenden Elemente können dann zur Erfassung des Fingerabdruckes 10 einzelne lichtempfindliche Elemente der Lage 1 in die Auswerteschaltung 7 ausgelesen werden.

## Patentansprüche

1. Vorrichtung zum Erfassen eines Fingerabdruckes mit einer lichtdurchlässigen, eine Fingerauflage bildenden Decklage und einer lichtemittierenden Lage mit einer photoaktiven Schicht, wobei zwischen der lichtdurchlässigen, eine Fingerauflage bildenden Decklage und der lichtemittierenden Lage eine Lage lichtempfindlicher Elemente in einer Matrixanordnung vorgesehen ist, die Vorrichtung ferner mit
einer an die lichtempfindlichen Elemente angeschlossenen Auswerteschaltung, **dadurch gekennzeichnet, daß** die Lage (1) lichtempfindlicher Elemente eine lichtdurchlässige, photoaktive Schicht (2) auf der Basis organischer Halbleiter zwischen zwei lichtdurchlässigen Elektrodenschichten (3, 4) aus einander kreuzenden Leiterbahnen (5, 6) aufweist
und dass die photoaktive Schicht der Lage (1) lichtempfindlicher Elemente und die photoaktive Schicht der lichtemittierenden Lage (9), zwischen sich eine gemeinsame Elektrodenschicht aufweisen.

2. Vorrichtung nach Anspruch 1, **dadurch gekennzeichnet, daß** die lichtemittierende Lage (9) in einzelne je für sich ansteuerbare Bereiche unterteilt ist.

3. Vorrichtung nach Anspruch 2, **dadurch gekennzeichnet, daß** die lichtemittierende Lage (9) eine photoaktive Schicht auf der Basis eines organischen Halbleiters zwischen zwei Elektrodenschichten aus einander kreuzenden Leiterbahnen aufweist, von denen die Elektrodenschicht zwischen der photoaktiven Schicht und der Lage (1) lichtempfindlicher Elemente zumindest bereichsweise lichtdurchlässig ist.

4. Vorrichtung nach einem der Ansprüche 1 bis 3, **dadurch gekennzeichnet, daß** die photoaktive Schicht (2) der Lage (1) lichtempfindlicher Elemente über eine Steuereinrichtung in zeitlicher Abhängigkeit von der Ansteuerung der lichtemittierenden Lage (9) ansteuerbar ist.

## Claims

1. A device for recording a fingerprint, having a translucent top layer which forms a finger rest and a light-emitting layer with a photoactive layer, wherein a layer of light-sensitive elements is provided in a matrix arrangement between the translucent top layer which forms a finger rest and the light-emitting layer, the device furthermore having an evaluation circuit connected to the light-sensitive elements, **characterized in that** the layer (1) of light-sensitive elements has a translucent, photoactive layer (2) based on organic semiconductors between two translucent electrode layers (3, 4) consisting of intersecting strip conductors (5, 6)
and **in that** the photoactive layer of the layer (1) of light-sensitive elements and the photoactive layer of the light-emitting layer (9) have between them a common electrode layer.

2. The device according to claim 1, **characterized in that** the light-emitting layer (9) is divided into individual regions, each of which can be activated independently.

3. The device according to claim 2, **characterized in that** the light-emitting layer (9) has a photoactive layer based on an organic semiconductor between two electrode layers consisting of intersecting strip conductors, of which the electrode layer between the photoactive layer and the layer (1) of light-sensitive elements is translucent at least in certain regions.

4. The device according to one of claims 1 to 3, **characterized in that** the photoactive layer (2) of the layer (1) of light-emitting elements can be activated via a control means as a function with respect to time of the activation of the light-emitting layer (9).

## Revendications

1. Dispositif pour détecter une empreinte digitale, pourvu d'une couche de recouvrement laissant passer la lumière formant appui pour doigt et d'une couche électroluminescente avec une strate photoactive, dans lequel est prévue, entre la couche de recouvrement laissant passer la lumière formant appui pour doigt et la couche électroluminescente, une couche d'éléments photosensibles agencés en une matrice, le dispositif comprenant en outre un circuit d'analyse relié aux éléments photosensibles, **caractérisés en ce que** la couche (1) d'éléments photosensibles présente une strate photoactive (2) laissant passer la lumière à base de semi-conducteur organique entre deux couches d'électrode (3, 4) laissant passer la lumière et constituées de pistes conductrices entrecroisées (5, 6),
et **en ce que** la strate photoactive de la couche (1) d'éléments photosensibles et la strate photoactive de la couche électroluminescente (9) présentent entre celles-ci une strates d'électrode commune.

2. Dispositif selon la revendication 1, **caractérisé en ce que** la couche électroluminescente (9) est divisée en zones individuelles pouvant être commandées séparément.

3. Dispositif selon la revendication 2, **caractérisé en ce que** la couche électroluminescente (9) comprend une strate photosensible à base d'un semi-conducteur organique entre deux strates d'électrode constituées de pistes conductrices entrecroisées, parmi lesquelles la strate d'électrode entre la strate photoactive et la couche (1) d'éléments photosensibles laisse passer la lumière au moins par endroits.

4. Dispositif selon l'une quelconque des revendications 1 à 3, **caractérisé en ce que** la strate photoactive (2) de la couche (1) d'éléments photosensibles peut être commandée par l'intermédiaire d'un dispositif de commande en synchronisme avec la commande de la couche électroluminescente (9).
